# EUROPEAN PATENT SPECIFICATION

(11) **EP 1 689 339 B1**
(45) Date of publication and mention of the grant of the patent: **16.09.2009**
(21) Application number: 04800349.5
(22) Date of filing: 18.11.2004
(51) Int. Cl.: A61F 13/15

(54) **ABSORBENT ARTICLE FOR NEWBORNS**
SAUGFÄHIGER ARTIKEL FÜR NEUGEBORENE
ARTICLE ABSORBANT POUR NOUVEAU-NES

(30) Priority: 20.11.2003 SE 0303068
(43) Date of publication of application: 16.08.2006
(73) Proprietor: SCA Hygiene Products AB, 405 03 Göteborg (SE)
(72) Inventor: GANDEMO, Tomas, S-436 38 Askim (SE); JUUL-JOHANSSON, Marianne, S-430 63 Hind s (SE)
(74) Representative: Romare, Laila Anette
(86) International application number: PCT/SE2004/001687
(87) International publication number: WO 2005/048900

(56) References cited:
- WO-A1-92/09254
- WO-A1-03/009792
- DE-A1- 2 951 399
- DE-A1- 19 721 271
- US-A- 4 230 113
- US-A- 4 675 015
- US-A- 4 769 023

## Description

### TECHNICAL FIELD

The invention relates to a disposable diaper for newborns. The diaper has a longitudinal direction and a transverse direction and comprises an upper liquid-permeable covering layer, a lower backing layer and an absorbent body arranged between the covering layer and the backing layer. The diaper also has a front end portion intended to be oriented forwards during use, the front end portion comprising a front transverse edge extending in the transverse direction of the diaper. The diaper also has a rear end portion intended to be oriented backwards during use, and a crotch portion arranged between the end portions. Fastening tabs are connected to the rear end portion. A fixing location is arranged for fixing the fastening tabs below the umbilical cord of the infant, the fixing location being arranged on the backing layer between the front transverse edge and the crotch portion.

### BACKGROUND ART

Specially designed absorbent diapers for newborns, who have not yet lost their umbilical cord, usually have cutouts or openings arranged at the front transverse edge of the diaper. In this connection, the cutout or opening is arranged so that the umbilical cord of the infant does not come into contact with the absorbent article.

Document DE 295 13 199 U1 describes an article which is manufactured with a cutout in order to leave the umbilical cord free.

Patent US 4,769,023 describes a conventional all-in-one diaper which is provided with a removable part in the front waist portion of the diaper. In this connection, an area is delimited by a perforation, and the perforated area can be separated from the rest of the diaper.

In the patent US 4,675,015, the diaper is provided with a perforated cross in the front waist portion. The perforations in the cross can be broken open, a hole through the diaper being formed. The umbilical cord is guided through the hole when the diaper is fitted on the infant.

One problem with diapers which comprise cutouts or openings is that the umbilical cord is not covered by the diaper and therefore has to be protected by some other form of auxiliary means. There may also be problems with the umbilical cord soiling the bed or the clothes of the infant or other places which come into contact with the umbilical cord.

There are diapers with fastening tabs which comprise more than one fixing location in the front portion for receiving the fastening tabs. In this connection, a first fixing location is arranged adjacent to the front transverse edge of the diaper, and other fixing locations are arranged between the first fixing location and the crotch portion of the diaper. The effective length of the diaper, that is to say the distance in the longitudinal direction of the diaper between the fastening tabs arranged in the rear portion of the diaper and the fixing location used, depends on which fixing location is used. The maximum effective diaper length is obtained when the first fixing location close to the front transverse edge of the diaper is used, while the minimum effective diaper length is obtained when the fastening tabs of the diaper are fixed to the fixing location arranged next to the crotch portion of the diaper. These diapers are fixed around the waist of the infant, the purpose of the various fixing locations being to adapt the size of the diapers according to the pelvis size of the infant as the infant grows.

Patent application WO 92/09254 describes a diaper which comprises alternative fixing surfaces arranged on the front portion of the diaper for fixing the fastening tabs arranged adjacent to the rear portion of the diaper. In this connection, a first fixing surface is arranged parallel and adjacent to the front transverse edge of the diaper, and a second fixing surface is arranged closer to the crotch portion of the diaper. The size of the diaper is adapted by the first fixing surface being used for larger infants with a larger pelvis size and the second fixing surface being used when the infant has a smaller pelvis size. The application also describes how the front part of the diaper can be folded in the transverse direction in order to make the diaper shorter. Irrespective of which fixing surface is used, however, the diaper is fixed around the waist of the infant.

For various reasons, many parents opt not to make use of special diapers for the short time the infant still has its umbilical cord but instead use ordinary baby diapers. So as not to risk damaging the umbilical cord of the infant, the parent fitting the diaper on the infant is in this connection apt to be extremely careful and tighten the diaper only loosely around the waist of the infant. Such fitting of a diaper certainly means that the umbilical cord is carefully protected but also means that the diaper fits loosely around the legs of the infant, which increases the risk of both faecal matter and urine leaking from the diaper.

A need therefore remains for a better functioning diaper for infants who have not yet lost their umbilical cord.

### DISCLOSURE OF INVENTION

With the present invention, however, a disposable diaper of the kind referred to in the introduction has been produced, which disposable diaper essentially eliminates the problems associated with previously known such disposable diapers.

In this connection, a disposable diaper for newborns made according to the invention is characterized mainly in that the fixing location is arranged so that the minimum distance between the fixing location and the front transverse edge of the diaper exceeds 4 centimetres, and that the disposable diaper comprises means for indicating the position of the fixing location.

According to one embodiment of the disposable diaper, the means for indicating the fixing location is arranged so that a minimum distance from the edge of the fastening tabs that is oriented towards the front transverse edge of the diaper to the front transverse edge of the diaper of 4 to 10 centimetres is obtained when the fastening tabs are fixed.

According to one embodiment of the disposable diaper, the means for indicating the fixing location is arranged so that a minimum distance from the edge of the fastening tabs to the front transverse edge of the diaper of 6 to 9 centimetres is obtained when the fastening tabs are fixed.

According to an alternative embodiment of the disposable diaper, the means for indicating the position of the fixing location consists of a colour marking.

According to another embodiment, the colour marking is printed directly on the backing layer.

According to a further embodiment, the means for indicating the position of the fixing location consists of an area with a visually differing surface texture in relation to the texture of the backing layer.

According to another embodiment of the disposable diaper, the means for indicating the position of the fixing location consists of the fixing location itself.

According to one embodiment of the disposable diaper, the means for indicating the position of the fixing location consists of an extra, separate, material piece.

According to one embodiment of the disposable diaper, the extra material piece comprises a touch-and-close surface of the hook type, that is to say with hook elements of some type, or of the loop type, that is to say with loop elements of some type.

According to another embodiment, the extra material piece consists of a plastic film.

According to one embodiment, the diaper comprises a second fixing location arranged on the backing layer at the front transverse edge of the diaper for fixing the fastening tabs around the waist of the infant.

### BRIEF DESCRIPTION OF FIGURES

The invention will be described in greater detail below with reference to the figures shown in the accompanying drawings.
- Figure 1: shows a disposable diaper comprising means for indicating a second receiving area according to the invention in a folded-out state from the side intended to face the wearer during use.
- Figure 2: shows a disposable diaper comprising means for indicating a second receiving area according to the invention in a folded-out state from the side intended to face away from the wearer during use.
- Figure 3: shows a disposable diaper according to one embodiment of the invention during fitting on an infant who has lost his/her umbilical cord.
- Figure 4: shows a disposable diaper according to one embodiment of the invention during fitting on an infant who has not lost his/her umbilical cord.
- Figure 5: shows a disposable diaper according to one embodiment of the invention during fitting on an infant who has not lost his/her umbilical cord.

### DESCRIPTION OF ILLUSTRATIVE EMBODIMENTS

The baby diaper for newborns shown in the figures comprises a securing system comprising a fixing location 34 on the front end portion 21 of the diaper 1 for fixing the fastening tabs 26 of the diaper 1. The fixing location 34 is specially adapted for infants who have not yet lost their umbilical cord.

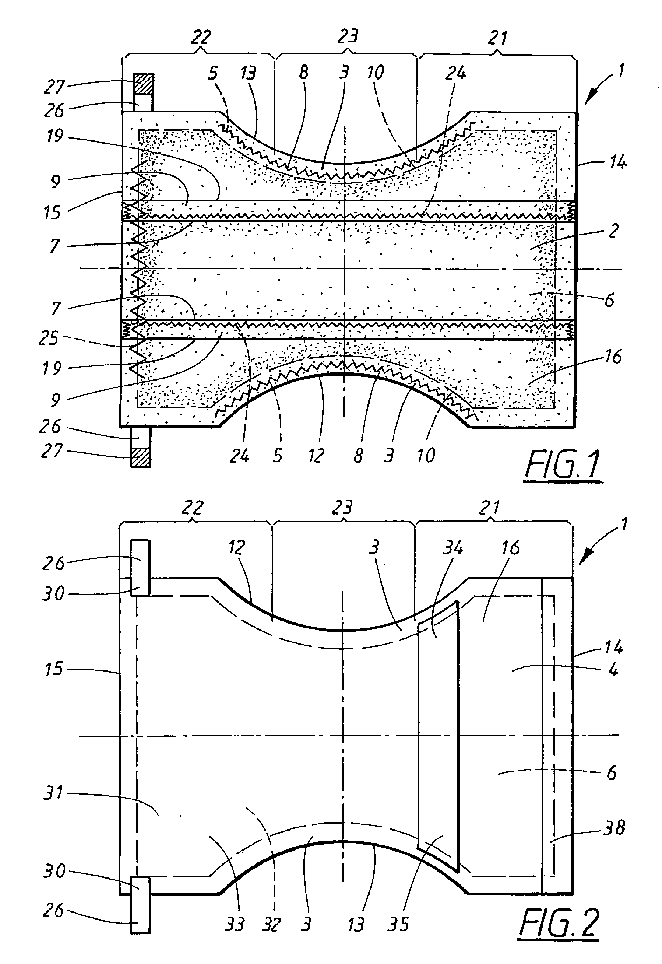

The first embodiment shown in Figure 1 and in Figure 2 concerns a baby diaper 1 which is suitable both for infants who have not yet lost their umbilical cord and for infants which have grown a little bigger and lost their umbilical cord. In this connection, the diaper 1 comprises two fixing locations 34, 38, one fixing location 38 being intended for fixing the diaper 1 around the waist of the infant, and the other fixing location 34 being intended for fixing the diaper 1 below the waist of the infant.

The invention relates to diapers 1 for newborn infants with a maximum weight of 6 kilograms and preferably with a weight of 3 to 6 kilograms. The diapers 1 therefore have a total length of less than 40 centimetres.

The diaper 1 is essentially hourglass-shaped and in this connection has longitudinal edges 12, 13, a front transverse edge 14 and a rear transverse edge 15, and also a front and a rear end portion 21, 22 and a narrower crotch portion 23 located between the end portions 21, 22. During use, the crotch portion 23 is intended to be located in the narrowest region between the thighs of the infant.

During use of the diaper 1, the front part of the crotch portion 23 and the front end portion 21 function mainly as a receiving area for urine, while the rear part of the crotch portion 23 and the rear end portion 22 function mainly as a receiving area for faecal matter.

The baby diaper 1 comprises a covering layer 16 comprising a liquid-permeable covering layer 2, arranged over that surface on the diaper 1 which is intended to face the infant during use, and a backing layer 4 arranged over that surface on the article which is intended to face away from the infant during use, an absorbent body 6 enclosed between the liquid-permeable covering layer 2 and the backing layer 4, and side flaps 3 arranged outside the absorbent body 6.

The liquid-permeable covering layer 2 extends outside the absorbent body 6 along the entire periphery of the absorbent body 6. The liquid-permeable covering layer 2 can consist of any material suitable for the purpose. Examples of common liquid-permeable covering materials are non-woven textile materials, perforated plastic films, nets made of plastic or textile, and liquid-permeable foam layers. Liquid-permeable covering materials which consist of continuous thin fibres which extend mainly in the longitudinal or transverse direction of the article also exist. Laminates consisting of two or more of the possible covering materials mentioned above are also common, as are coverings consisting of different materials within different parts of the surface.

Disposable diapers 1 comprising absorbent bodies 6 which have particularly high strength and wear-resistance can even function without any extra liquid-permeable covering layer being required on that side of the diaper which faces the infant during use.

The backing layer 4 consists of a laminate 31 comprising a liquid-impermeable plastic film 32 arranged against the absorbent body 6 and a non-woven layer 33, the non-woven layer 33 being arranged away from the absorbent body 6 so that the outside of the diaper 1 is more cloth-like during use of the diaper 1. The backing layer 4 extends outside the absorbent body 6 along the entire periphery of the absorbent body 6.

Backing layers 4 on absorbent articles usually consist of liquid-impermeable plastic films or of laminates which include liquid-impermeable plastic films, but other types of liquid-impermeable backing layer are also found. Examples of alternative types of liquid-impermeable material are non-woven materials which have been made liquid-impermeable, liquid-impermeable foam layers, liquid-impermeable adhesive or the like. It is also common today for the backing layer 4 to consist of a vapour-permeable plastic film, or of a laminate which includes a vapour-permeable plastic film as a liquid barrier. The vapour-permeable plastic film must of course be liquid-impermeable in order to prevent penetration of liquid from the absorbent body.

When the backing layer 4 of the diaper 1 comprises a non-woven layer 33 as in the embodiment described, the non-woven layer 33 can be designed so that it also functions as a receiving layer for a touch-and-close material of the male type, that is to say with hook elements of some type. In this connection, the non-woven layer 33 comprises closed loops or the like.

In alternative embodiments, the non-woven layer 33 can be designed so that it is not capable of interacting with a touch-and-close material of the male type, special receiving materials then having to be arranged on the non-woven layer 33.

The liquid-permeable covering layer 2 and the backing layer 4 are interconnected outside the absorbent body 6 along the entire periphery of the absorbent body 6. The layers 2, 4 can be interconnected in a number of different ways. Examples of common connecting methods are gluing, thermal welding, ultrasonic welding or the like.

Elastic means 5 are arranged outside the absorbent body 6 in those parts of the side flaps 3 of the disposable diaper 1 which essentially run in the longitudinal direction of the diaper 1. The elastic means 5 function as leg elastic and have the task of preventing liquid and motions leaking out through the side edges 12, 13 running in the longitudinal direction and in this way, together with surrounding layers, form outer liquid barriers 8. The elastic means 5 consist of one or more elastic threads which have, in a stretched state, been applied between the liquid-permeable covering layer 2 and the backing layer 4, at least in the crotch portion 23 of the diaper 1. The elastic means 5 are connected to the backing layer 4 and the covering layer 2 by gluing, ultrasonic welding or the like.

In alternative embodiments, the elastic means can be arranged on the side of the side flaps 3 which is intended to face the wearer during use, or on the opposite side of the side flaps, and are then of course connected to only the covering layer 2 or, respectively, the backing layer 4.

In alternative embodiments, the elastic means can consist of elastic band materials made of, for example, foam material.

The hourglass-shaped absorbent body 6 can be constructed from one or more layers of cellulose fluff pulp. In this connection, the cellulose fluff pulp can be mixed with fibres or particles of a highly absorbent polymer material of the kind which, during absorption, chemically binds great quantities of liquid while forming a liquid-containing gel. The absorbent body 6 can also comprise highly absorbent polymer material arranged in a layer inside the absorbent body or adjacent to the surface or surfaces of the absorbent body. The absorbent body 6 can also include further components for improving the properties of the absorbent body 6. Examples of such components are bonding fibres, various types of liquid-spreading layer or fibre, shape-stabilizing components, strengthening fibres or the like. The absorbent body 6 can of course also consist of other types of absorbent material, such as absorbent non-woven materials, absorbent foams, textile materials, peat or mixtures of different kinds of absorbent material.

Special layers for rapidly receiving great quantities of liquid and temporarily retaining this liquid in order then to transfer the temporarily stored liquid to other parts of the absorbent body 6 can also be included in diapers of the type indicated. Such receiving layers are then normally arranged between the liquid-permeable covering layer 2 and absorbent body 6 of the baby diaper 1. No receiving layer is illustrated in any of the figures.

In order further to prevent liquid or faecal matter leaking out over the side edges 12, 13 of the baby diaper 1, the diaper 1 is provided with inner side leakage barriers 9 on the side which is intended to face the infant during use. The inner side leakage barriers 9 are arranged adjacent to the longitudinal edges 10 of the absorbent body 6 and extend essentially in the longitudinal direction of the diaper 1. The side leakage barriers 9 are made from separate material strips folded in two, the fold edges 7 constituting the ridges of the side leakage barriers 9. The respective legs of the material strips folded in two are fixed to the covering layer 2 and constitute the fixed edges 19 of the side leakage barriers.

In the front end portion 21 and rear end portion 22 of the baby diaper 1, the inner side leakage barriers 9 are folded down and connected to the covering layer 2 over their entire widths.

The inner side leakage barriers 9 comprise elastic elements 24 connected to the inner side leakage barriers 9 in a pretensioned state. The elastic elements 24 are preferably arranged adjacent to the free, unfixed edges of the inner side leakage barriers 9. When the pretensioned elastic elements 24 are released, they contract together with the free edges of the inner side leakage barriers 9, the inner side leakage barriers 9 being brought into an erect configuration away from the liquid-permeable covering layer 2 in the crotch portion 23 of the diaper 1, where the side leakage barriers 9 are connected to the covering layer 2 only at their respective free edges.

The rear and/or front end portions 22, 21 of the baby diaper 1 can also be provided with what is known as waist elastic 25 which consists of elastic means arranged along the front transverse edge 14 and/or the rear transverse edge 15 of the diaper 1 so that the diaper encloses the waist of the wearer gently and flexibly. In the present illustrative embodiment, only the rear end portion 22 of the diaper 1 is provided with waist elastic 25 in the form of a thin strip of an elastic foam material which is attached by glue between the backing layer 4 and the liquid-permeable surface layer 2. The waist elastic 25 is applied in a stretched state between the layers in order to bring about a holding-together force which stretches the diaper 1 around the waist of the wearer.

Arranged on the rear end portion 22 are two soft and inelastic fastening tabs 26 for fixing the baby diaper 1 around the waist of the infant, one fastening tab 26 in this connection being arranged on each side portion of the rear end portion 22. The fastening tabs 26 are suitably made from a very soft and inelastic material, for example from a single non-woven layer or a laminate. During use, the fastening tabs 26 connect the rear end portion 22 to the front end portion 21.

The fastening tabs 26 comprise fixing elements 27, the fixing elements 27 preferably consisting of a male part of a touch-and-close material and being attached to the fastening tabs 26 by glue or the like. The fixing elements 27 are in this connection arranged on that side of the respective fastening tab 26 which faces that surface on the front end portion 21 which faces away from the infant during use. In alternative embodiments, the fastening tabs 26 can be elastic.

The fastening tabs 26 are connected to the rear end portion 22 in connecting areas 30 which are positioned in the areas of the rear end portion 22 which lie at the side edges 12, 13 running in the longitudinal direction. The connecting areas 30 consist of parts of the fastening tabs 26 and the parts of the rear end portion 22 which are interconnected. In alternative embodiments, the fixing elements 27 of the fastening tabs 26 can consist of female parts of a touch-and-close material, pressure-sensitive adhesive or the like.

When the diaper 1 is fitted around the waist of an infant, the rear end portion 22 is interconnected with the front end portion 21 by the fixing elements 27 of the fastening tabs 26 being connected to the front end portion 21. As the diaper 1, according to the first embodiment, has a backing layer 4 which comprises a non-woven layer 33 functioning as a receiving layer for a touch-and-close material of the male type, the fixing element 27 can be attached to the backing layer 4 of the diaper 1 anywhere.

For alternative embodiments where the backing layer 4 of the diaper 1 is not adapted to interact with the fixing elements 27 of the fastening tabs 26, one or more special fixing locations 34, 38 are arranged on the backing layer 4 of the diaper 1 in the front end portion 21. In this connection, the special fixing locations 34, 38 consist of separate material pieces of suitable shape and size, the material pieces consisting of material which can be connected to the fixing elements 27 of the fastening tabs 26. The separate material pieces are applied to the backing layer 4 by means of gluing, thermal welding, ultrasonic welding or the like. The number and positioning of the fixing locations 34, 38 on the front end portion can vary, but it is most usual for a rectangular fixing location 38 for fixing the diaper around the waist of the infant to be arranged parallel and adjacent to the front transverse edge 14 of the diaper 1, the fixing location 38 extending over the entire width of the diaper 1.

Variants of diaper 1 where the fixing location 38 for waist fixing consists of two receiving surfaces arranged in the front end portion 21 are also found. The receiving surfaces are then arranged in respective front corners of the diaper, that is to say adjacent to the front transverse edge 14 of the diaper and the respective longitudinal edge 12, 13.

Diapers 1 comprising fixing elements 27 of adhesive type comprise fixing locations 34, 38 which are suitable for receiving an adhesive fixing element 27, such as a plastic surface or the like. The fixing locations 34, 38 then usually consist of separate material pieces of plastic film of suitable shape and size applied to the backing layer 4 in suitable locations by gluing, thermal welding, ultrasonic welding or the like.

It is also common for diapers 1 equipped with adhesive fixing elements 27 to have backing layers 4 which consist only of plastic film or the like; the adhesive fixing element 27 can then be attached directly to the backing layer 4 of the diaper 1 anywhere on the backing layer 4. These diapers 1 have in principle an infinite number of receiving surfaces for the fastening tabs 26.

When diapers 1 are to be fitted on newborn infants who have not yet lost their umbilical cord, there is often a conflict between protection of the umbilical cord of the infant and satisfactory fixing of the diaper 1 on the infant. Satisfactory fixing of the diaper 1 on the infant is important in order that the diaper 1 will fit correctly so that optimum functioning of the diaper 1 is obtained. A correctly positioned diaper 1 means inter alia that the absorbent body is correctly positioned in relation to the urine opening and anus of the infant. A correctly positioned diaper 1 also means that its outer and inner leakage barriers 8, 9 occupy a correct position in relation to the body of the infant with a minimum of gaps and creases where urine and faecal matter can pass the barriers and cause leakage. An important element in achieving satisfactory fixing is tightening the diaper 1 sufficiently firmly around the waist of the infant.

On the other hand, protection of the umbilical cord means that the diaper 1 should not be tightened too firmly around the waist of the infant so that the pressure against the umbilical cord is not too great. After being used for a while, a diaper 1 which is fixed loosely around the waist of the infant often becomes incorrectly positioned on the body of the infant, which means that the advantages mentioned above are lost. There is also a considerably increased risk of urine and faecal matter leaking out via the waist opening of a diaper which has not been tightened firmly enough.

Tests have nevertheless shown that both satisfactory fixing of a diaper 1 and protection of the umbilical cord can be achieved, which a small number of parents of newborn infants also appear to have discovered.

The principle for fixing a diaper 1 on an infant who has not lost his/her umbilical cord is based on attaching the fastening tabs 26 of the diaper 1 to a fixing location 34 located on the front end portion 21 considerably closer to the crotch portion 23 of the diaper. In this connection, the fastening tabs 26 are fixed so close to the crotch portion 23 that the fixing lies below the umbilical cord of the infant; the diaper 1 can then be tightened normally around the infant. Normal tightening of the diaper 1 around the infant means full functioning of barriers 8, 9 and correct positioning of the absorbent body 6 in relation to the body of the infant, without excessive pressure against the umbilical cord occurring.

The fixing location 34 should be located at a distance from the front transverse edge 14 of the diaper 1 of 4 to 10 cm, preferably at a distance of 6 to 9 cm. When the diaper 1 is fixed in this way, the front part of the diaper 1, that is to say the part between the fixing location 34 and the front transverse edge 14, will bear against the abdomen and umbilical cord of the infant with only a light pressure. The front part of the diaper 1 will then function as protection for the umbilical cord, urine or faecal matter being prevented from reaching the umbilical cord. The front part of the diaper 1 will also function as protection against the umbilical cord of the infant soiling surrounding clothes and bedclothes.

A small number of parents have discovered that it is expedient to fix a diaper 1 according to this alternative method on their newborn infants and thus achieve the advantages described above.

It is a common feature of the diapers 1 which were used in tests and by the parents who discovered fixing the diapers in the alternative way described that they have a backing layer 4 to which the fixing elements 27 of the fastening tabs 26 can be fixed directly, anywhere on the surface of the backing layer 4. In this connection, the diapers 1 were provided with fixing elements 27 on the fastening tabs 26 made of touch-and-close material of the male type and with a backing layer 4 comprising a non-woven layer 33 comprising loops to which the fixing elements 27 can be fixed. Alternatively, the diapers 1 were provided with fastening tabs comprising adhesive fixing elements 27 and a plastic film as the backing layer 4.

A baby diaper 1 according to the invention is characterized mainly in that it comprises means 35 for indicating the fixing location 34.

As the method of fixing a diaper 1 described above is appropriate only during the short time the infant still has his/her umbilical cord, it is only a small number of parents who see and test the possibility of fixing the diaper in this way. It is therefore important that new parents are given a visual signal of the possibility of fixing the diaper 1 so that the umbilical cord is protected and so that functioning is maintained as far as leakproofness is concerned. In accordance with the invention, the signal is given to the parents in the form of an indication on the backing layer 4 of the diaper of the position of the fixing location 34 on the diaper 1.

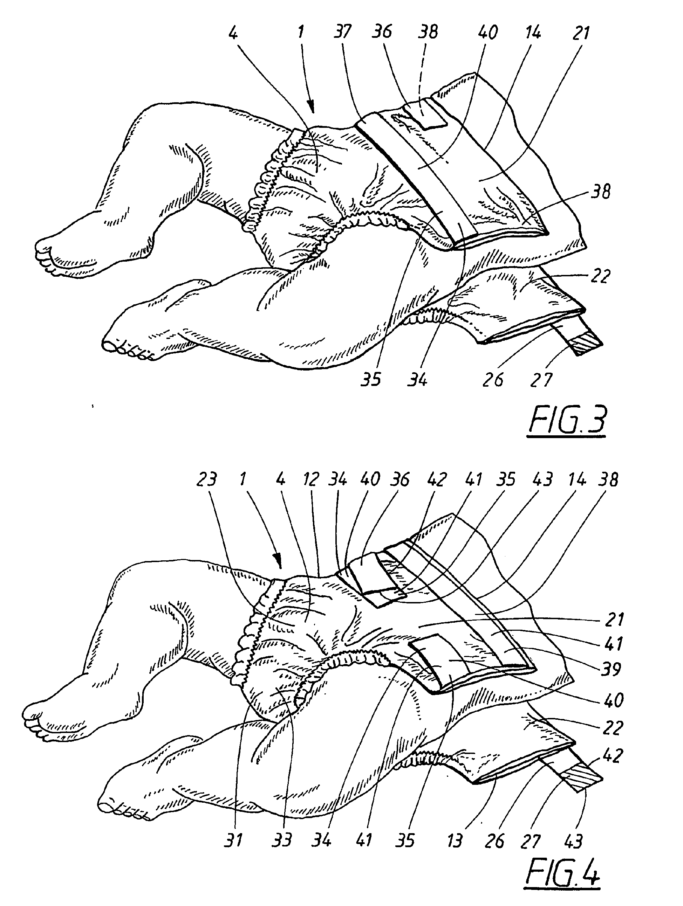

In Figures 2 and 3, the means 35 for indicating the fixing location 34 consists of an oblong colour marking which extends in the transverse direction of the diaper 1, essentially over the entire width of the diaper 1.

It is also conceivable to design the means 35 for indicating the fixing location 34 in another way; conceivable designs are, for example, lines of text, symbols, special material layers arranged at the alternative, second fixing location 34 or the like.

Another possible embodiment is for the means 35 for indicating the fixing location 34 to consist of a visible material layer which constitutes a particularly suitable receiving material for the fixing elements 27 of the fastening tabs 26. For example, it is suitable to select a clearly visible touch-and-close material if the fixing element 27 is of the touch-and-close type, and a clearly visible plastic film if the fixing element is an adhesive fixing element 27. These types of means 35 for indicating the fixing location 34 are suitable mainly for diapers 1 which comprise fixing elements 27 which cannot be fixed directly to the backing layer 4 of the diaper 1.

Such diapers also comprise special receiving materials for conventional waist fixing, the fastening tabs 26 being fixed to the front end portion 21 of the diaper 1 next to the front transverse edge 14 of the diaper 1.

Figure 3 shows a diaper 1 in the course of being fitted around the waist on an infant who has lost his/her umbilical cord, the rear end portion 22 of the diaper 1 being connected to the front end portion 21 close to the front transverse edge 14 of the diaper 1. In this respect, the fastening tab 36 on one side has already been connected to the front end portion 21 of the diaper 1, while the fastening tab 26 on the other side has not yet been fixed to the front end portion 21 of the diaper.

The backing layer 4 of the diaper 1 consists of a liquid-impermeable plastic film. The fixing elements 27 of the fastening tabs 26, 36 consist of adhesive surfaces which can be attached to the backing layer 4 anywhere, the diaper 1 having no visual indication of the fixing location 38 next to the front transverse edge 14. The position of the fixing location 34 instead is indicated by means of an oblong coloured surface 37 which extends across the entire width of the diaper and constitutes the means 35 for indicating the fixing location 34. The oblong coloured surface 37 consists of a coloured plastic strip 40. In alternative embodiments, the coloured oblong surface 37 can consist of a colour which is printed directly on the backing layer 4 or the like. The whole of the oblong surface 37 does not necessarily have to be coloured, but it can suffice for only the outer delimiting lines of the surface 37 to be indicated. Decorative patterns or the like are also conceivable in order to indicate visually the oblong surface 37. The coloured oblong surface 37 is located at a distance from the front transverse edge 14 of the diaper 1 of 4 to 10 cm, preferably at a distance of 6 to 9 cm.

Figure 4 shows an alternative embodiment of a diaper 1 in the course of being fitted on a newborn infant who still has his/her umbilical cord. In this connection, fitting has advanced so far that the rear end portion 22 has been connected to the front end portion 21 on one side of the diaper 1, the fastening tab 36 having been connected to the front end portion 21. In this respect, the fastening tab 36 has been connected to the front end portion 21 so that the edge 42 of the fastening tab 36, that is to say the edge 42 which is oriented towards the front transverse edge 14 of the diaper 1, has a minimum distance of 4 centimetres from the front transverse edge 14 of the diaper 1 in the longitudinal direction of the diaper 1. In this respect, the minimum distance is measured from a point on the edge 42 of the fastening tab 36 located 0.5 centimetres in on the fastening tab 36 from its free end 43.

The fastening tab 26 on the other side has not yet been fixed to the front end portion 21 of the diaper.

As the infant has not yet lost his/her umbilical cord, the fastening tab 36 has, in order to protect the umbilical cord, been fixed to the fixing location 34 of the diaper 1 which is arranged between the fixing location 38 of the diaper 1 for waist fixing and the crotch portion 23. The other fastening tab 26, which has not yet been fixed, will in the next fitting stage be fixed to the second, alternative fixing location 34.

The backing layer 4 of the diaper 1 consists of a laminate 31 which comprises a non-woven layer 33 arranged on the outside of the diaper 1. The non-woven layer 33 is not designed so that the fixing elements 27 of the fastening tabs 26, 36 can be fixed directly to the non-woven layer 33, for which reason special receiving surfaces 39, 40 are arranged on the backing layer 4 of the diaper 1.

One receiving surface 39 is arranged parallel and adjacent to the front transverse edge 14 of the diaper 1 and is intended for conventional fixing of the diaper 1 on an infant who has already lost his/her umbilical cord. The receiving surface 39 extends over the entire width of the diaper 1.

The alternative fixing location 34 of the diaper 1 comprises two receiving surfaces 40, one receiving surface 40 being arranged so as to be connected to one fastening tab 36, and the other receiving surface 40 being arranged so as to be connected to the other fastening tab 26. The receiving surfaces 39, 40 consist of separate material strips 41 of touch-and-close material of the female type. The receiving surfaces 40 extend from the respective longitudinal edge 12, 13 towards the longitudinal centre line of the diaper 1. The fixing elements 27 of the fastening tabs 26, 36 consist of touch-and-close material of the male type. The material strips 41 are visible and therefore also constitute the means 35 for indicating the alternative fixing location 34.

In order to make the means 35 for indicating the fixing location 34 even more visible, it is also possible to select the material strips 41 of touch-and-close material in a different colour from the backing layer 4 or the like.

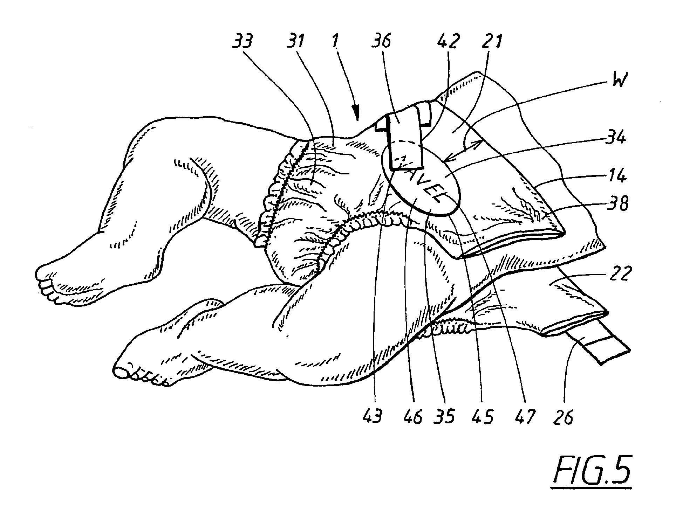

Figure 5 shows another alternative embodiment of a diaper 1 in the course of being fitted on a newborn infant who still has his/her umbilical cord.

In this connection, fitting has advanced so far that the rear end portion 22 has been connected to the front end portion 21 on one side of the diaper 1, the fastening tab 36 having been connected to the front end portion 21. In this respect, the fastening tab 36 has been connected to the front end portion 21 so that the edge 42 of the fastening tab 36, that is to say the edge 42 which is oriented towards the front transverse edge 14 of the diaper 1, has a minimum distance of 4 centimetres from the front transverse edge 14 of the diaper 1 in the longitudinal direction of the diaper 1. In this respect, the minimum distance is measured from a point on the edge 42 of the fastening tab 36 located 0.5 centimetres in on the fastening tab 36 from its free end 43. The fastening tab 26 on the other side has not yet been fixed to the front end portion 21 of the diaper.

As the infant has not yet lost his/her umbilical cord, the fastening tab 36 has, in order to protect the umbilical cord, been fixed to the fixing location 34 of the diaper 1 arranged between the fixing location 38 of the diaper 1 for waist fixing and the crotch portion 23. The other fastening tab 26, which has not yet been fixed, will in the next fitting stage be fixed to the alternative, second fixing location 34.

The backing layer 4 of the diaper 1 consists of a laminate 31 comprising a non-woven layer 33 arranged on the outside of the diaper 1. The non-woven layer 33 is designed so that the fixing elements 27 of the fastening tabs 26, 36 can be fixed directly to the non-woven layer 33, anywhere on it. The backing layer 4 of the diaper 1 therefore has no specially arranged receiving surfaces for the fastening tabs 26, 36.

The diaper has no means for visual indication of the fixing location 38 for waist fixing next to the front transverse edge 14. The fixing location 34, for fixing the diaper 1 on an infant who still has his/her umbilical cord, on the other hand has means 35 for indicating this fixing location. In this connection, the means 35 for indicating the position of the fixing location 34 consists of an oval surface 45 which has a different colour in relation to the backing layer 4. The oval surface 45 is arranged essentially symmetrically around the longitudinal symmetry line of the diaper 1, the largest extent of the oval surface 45 being in the transverse direction of the diaper 1.

Alternative shapes of the indicating surface, such as a circle or a rectangle with rounded corners or the like, are of course also conceivable.

The oval indicating surface consists of a separate oval coloured material piece 46 which is applied under the non-woven layer 33 which constitutes the outermost part of the laminate 31 constituting the backing layer 4 of the diaper 1. The strength of the colour of the oval material piece 46 has been selected so that it is visible through the relatively transparent non-woven layer 33.

A line of text 47 which makes it still clearer that it is suitable to attach the fastening tabs 26, 36 of the diaper 1 in this area of the front end portion 21 of the diaper 1 when the infant still has his/her umbilical cord has also been printed on the oval material piece 46 in another colour which can be seen through the non-woven layer 33.

The minimum distance W between the oval material piece 46 and the front transverse edge 14 of the diaper 1 is 4 to 10 centimetres, preferably 6 to 9 centimetres. In this connection, the distance is measured from that point on the periphery of the oval material piece 46 which has the smallest perpendicular distance from the front transverse edge 14. The distance is measured when the diaper 1 is in a plane stretched-out state like that in which the diaper 1 is shown in Figures 1 and 2.

The invention also includes all conceivable combinations of the illustrative embodiments described.

Furthermore, the invention is not limited to the illustrative embodiments above, but can of course be applied to other embodiments within the scope of the patent claims below.

## Claims

1. Disposable diaper (1) for newborns, having a longitudinal direction and a transverse direction and comprising an upper liquid-permeable covering layer (2), a lower backing layer (4), an absorbent body (6) arranged between the covering layer (2) and the backing layer (4), a front end portion (21) intended to be oriented forwards during use comprising a front transverse edge (14) extending in the transverse direction of the diaper (1), a rear end portion (22) intended to be oriented backwards during use, and a crotch portion (23) arranged therebetween, fastening tabs (26, 36) connected to the rear end portion (22), and a fixing location (34) for fixing the fastening tabs (26, 36) below the umbilical cord of the infant, the fixing location (34) being arranged on the backing layer (4) between the front transverse edge (14) and the crotch portion (23), **characterized in that** the fixing location (34) is arranged so that the minimum distance (W) between the fixing location (34) and the front transverse edge (14) of the diaper (1) exceeds 4 centimetres, and that the disposable diaper (1) comprises means (35) for indicating the position of the fixing location (34).

2. Disposable diaper (1) according to Claim 1, **characterized** i n that the means (35) for indicating the fixing location (34) is arranged so that a minimum distance from the edge (42) of the fastening tabs (26, 36) that is oriented towards the front transverse edge (14) of the diaper (1) to the front transverse edge (14) of the diaper (1) of 4 to 10 centimetres is obtained when the fastening tabs (26, 36) are fixed.

3. Disposable diaper (1) according to Claim 2, **characterized in that** the means (35) for indicating the fixing location (34) is arranged so that a minimum distance from the edge (42) of the fastening tabs (26, 36) that is oriented towards the front transverse edge (14) of the diaper (1) to the front transverse edge (14) of the diaper (1) of 6 to 9 centimetres is obtained when the fastening tabs (26, 36) are fixed.

4. Disposable diaper (1) according to any of the preceding claims, **characterized in that** the means (35) for indicating the position of the fixing location (34) consists of a colour marking.

5. Disposable diaper (1) according to Claim 4, **characterized in that** the colour marking is printed directly on the backing layer (4).

6. Disposable diaper (1) according to any of Claims 1-3, **characterized in that** the means (35) for indicating the position of the fixing location (34) consists of an area with a visually differing surface texture in relation to the texture of the backing layer (4).

7. Disposable diaper (1) according to any one of the preceding claims, **characterized in that** the means (35) for indicating the position of the fixing location (34) constitutes the fixing location (34).

8. Disposable diaper (1) according to Claim 7, **characterized in that** the means (35) for indicating the position of the fixing location (34) consists of an extra material piece (40).

9. Disposable diaper (1) according to Claim 8, **characterized in that** the extra material piece (40) comprises a touch-and-close surface of the hook type or loop type.

10. Disposable diaper (1) according to Claim 8, **characterized in that** the extra material piece (40) consists of a plastic film.

11. Disposable diaper (1) according to any one of the preceding claims, **characterized in that** the diaper (1) comprises another fixing location (38) arranged on the backing layer (4) at the front transverse edge (14) of the diaper (1) for fixing the fastening tabs (26, 36) around the waist of the infant.

## Patentansprüche

1. Wegwerfwindel (1) für Neugeborene mit einer Längsrichtung und einer Querrichtung und umfassend eine obere flüssigkeitsdurchlässige Abdeckschicht (2), eine untere Stützschicht (4) und einen Absorbenskörper (6), der zwischen der Abdeckschicht (2) und der Stützschicht (4) angeordnet ist, einen Frontendabschnitt (21), der dafür gedacht ist, während der Verwendung nach vorne ausgerichtet zu sein, umfassend eine vordere Querkante (14), die sich in der Querrichtung der Windel (1) erstreckt, einen Rückendabschnitt (22), der dafür gedacht ist, während der Verwendung nach hinten ausgerichtet zu sein, und einen Schrittabschnitt (23), der dazwischen angeordnet ist, Befestigungslaschen (26, 36), die mit dem Rückendabschnitt (22) verbunden sind, und einen Befestigungsort (34) zum Befestigen der Befestigungslaschen (26, 36) unterhalb der Nabelschnur des Kinds, wobei der Befestigungsort (34) auf der Stützschicht (4) zwischen der vorderen Querkante (14) und dem Schrittabschnitt (23) angeordnet ist, **dadurch gekennzeichnet, dass** der Befestigungsort (34) so angeordnet ist, dass der minimale Abstand (W) zwischen dem Befestigungsort (34) und der vorderen Querkante (14) der Windel (1) 4 cm übersteigt und dass die Wegwerfwindel (1) ein Mittel (35) zum Anzeigen der Position des Befestigungsorts (34) aufweist.

2. Wegwerfwindel (1) nach Anspruch 1, **dadurch gekennzeichnet, dass** das Mittel (35) zum Anzeigen des Befestigungsorts (34) so angeordnet ist, dass ein minimaler Abstand von der Kante (42) der Befestigungslaschen (26, 36), die in Richtung der vorderen Querkante (14) der Windel (1) ausgerichtet ist, zu der vorderen Querkante (14) der Windel (1) von 4 bis 10 cm erhalten wird, wenn die Befestigungslaschen (26, 36) befestigt sind.

3. Wegwerfwindel (1) nach Anspruch 2, **dadurch gekennzeichnet, dass** das Mittel (35) zum Anzeigen des Befestigungsorts (34) so angeordnet ist, dass ein minimaler Abstand von der Kante (42) der Befestigungslaschen (26, 36), die in Richtung der vorderen Querkante (14) der Windel (1) ausgerichtet ist, zu der vorderen Querkante (14) der Windel (1) von 6 bis 9 cm erhalten wird, wenn die Befestigungslaschen (26, 36) befestigt sind.

4. Wegwerfwindel (1) nach einem der vorstehenden Ansprüche, **dadurch gekennzeichnet, dass** das Mittel (35) zum Anzeigen der Position des Befestigungsorts (34) aus einer Farbmarkierung besteht.

5. Wegwerfwindel (1) nach Anspruch 4, **dadurch gekennzeichnet, dass** die Farbmarkierung direkt auf die Stützschicht (4) gedruckt ist.

6. Wegwerfwindel (1) nach einem der Ansprüche 1 bis 3, **dadurch gekennzeichnet, dass** das Mittel (35) zum Anzeigen der Position des Befestigungsorts (34) aus einem Gebiet mit einer visuell abweichenden Oberflächentextur in Bezug auf die Textur der Stützschicht (4) besteht.

7. Wegwerfwindel (1) nach einem der vorstehenden Ansprüche, **dadurch gekennzeichnet, dass** das Mittel (35) zum Anzeigen der Position des Befestigungsorts (34) den Befestigungsort (34) bildet.

8. Wegwerfwindel (1) nach Anspruch 7, **dadurch gekennzeichnet, dass** das Mittel (35) zum Anzeigen der Position des Befestigungsorts (34) aus einem extra Materialstück (40) besteht.

9. Wegwerfwindel (1) nach Anspruch 8, **dadurch gekennzeichnet, dass** das extra Materialstück (40) eine Berühr-und-Schließoberfläche des Hakentyps oder Ösentyps aufweist.

10. Wegwerfwindel (1) nach Anspruch 8, **dadurch gekennzeichnet, dass** das extra Materialstück (40) aus einem Kunststofffilm besteht.

11. Wegwerfwindel (1) nach einem der vorstehenden Ansprüche, **dadurch gekennzeichnet, dass** die Windel (1) einen weiteren Befestigungsort (38) aufweist, der auf der Stützschicht (4) an der vorderen Querkante (14) der Windel (1) zum Befestigen der Befestigungslaschen (26, 36) um die Taille des Kinds angeordnet ist.

## Revendications

1. Couche jetable (1) pour nouveau-nés, présentant un sens longitudinal et un sens transversal et comprenant une couche de revêtement perméable au liquide supérieure (2), une couche de doublage inférieure (4), un corps absorbant (6) agencé entre la couche de revêtement (2) et la couche de doublage (4), une partie d'extrémité avant (21) destinée à être orientée vers l'avant au cours de l'utilisation comprenant un bord transversal avant (14) s'étendant dans le sens transversal de la couche (1), une partie d'extrémité arrière (22) destinée à être orientée vers l'arrière au cours de l'utilisation, et une partie d'entrejambe (23) agencée entre elles, des pattes de fixation (26, 36) reliées à la partie d'extrémité arrière (22) et un emplacement de fixation (34) pour fixer les pattes de fixation (26, 36) sous le cordon ombilical du nourrisson, l'emplacement de fixation (34) étant agencé sur la couche de doublage (4) entre le bord transversal avant (14) et la partie d'entrejambe (23), **caractérisée en ce que** l'emplacement de fixation (34) est agencé de telle sorte que la distance minimale (W) entre l'emplacement de fixation (34) et le bord transversal avant (14) de la couche (1) dépasse 4 centimètres et **en ce que** la couche jetable (1) comprend des moyens (35) pour indiquer la position de l'emplacement de fixation (34).

2. Couche jetable (1) selon la revendication 1, **caractérisée en ce que** les moyens (35) destinés à indiquer l'emplacement de fixation (34) sont agencés de telle sorte qu'une distance minimale entre le bord (42) des pattes de fixation (26, 36) qui est orienté vers le bord transversal avant (14) de la couche (1) et le bord transversal avant (14) de la couche (1) de 4 à 10 centimètres est obtenue lorsque les pattes de fixation (26, 36) sont fixées.

3. Couche jetable (1) selon la revendication 2, **caractérisée en ce que** les moyens (35) destinés à indiquer l'emplacement de fixation (34) sont agencés de telle sorte qu'une distance minimale entre le bord (42) des pattes de fixation (26, 36) qui est orienté vers le bord transversal avant (14) de la couche (1) et le bord transversal avant (14) de la couche (1) de 6 à 9 centimètres est obtenue lorsque les pattes de fixation (26, 36) sont fixées.

4. Couche jetable (1) selon l'une quelconque des revendications précédentes, **caractérisée en ce que** les moyens (35) destinés à indiquer la position de l'emplacement de fixation (34) comprennent un marquage de couleur.

5. Couche jetable (1) selon la revendication 4, **caractérisée en ce que** le marquage de couleur est imprimé directement sur la couche de doublage (4).

6. Couche jetable (1) selon l'une quelconque des revendications 1 à 3, **caractérisée en ce que** les moyens (35) destinés à indiquer la position de l'emplacement de fixation (34) comprennent une zone avec une texture de surface qui diffère visuellement de la texture de la couche de doublage (4).

7. Couche jetable (1) selon l'une quelconque des revendications précédentes, **caractérisée en ce que** les moyens (35) destinés à indiquer la position de l'emplacement de fixation (34) constituent l'emplacement de fixation (34).

8. Couche jetable (1) selon la revendication 7, **caractérisée en ce que** les moyens (35) destinés à indiquer la position de l'emplacement de fixation (34) comprennent un morceau de matière supplémentaire (40).

9. Couche jetable (1) selon la revendication 8, **caractérisée en ce que** le morceau de matière supplémentaire (40) comprend une surface accrochante de type crochet ou boucle.

10. Couche jetable (1) selon la revendication 8, **caractérisée en ce que** le morceau de matière supplémentaire (40) est un film en plastique.

11. Couche jetable (1) selon l'une quelconque des revendications précédentes, **caractérisée en ce que** la couche (1) comprend un autre emplacement de fixation (38) agencé sur la couche de doublage (4) au niveau du bord transversal avant (14) de la couche (1) pour fixer les pattes de fixation (26, 36) autour de la taille du nourrisson.
